# DEMANDE DE BREVET EUROPEEN

(11) **EP 2 332 544 A1**
(43) Date de publication de la demande: **15.06.2011**
(21) Numéro de dépôt: 09176661.8
(22) Date de dépôt: 20.11.2009
(51) Int. Cl.: A61K 31/551, A61K 31/536, A61K 31/505, A61K 31/513, A61K 31/7068, A61K 31/52, A61K 31/675, A61P 31/18

(54) **Nouvelle composition pharmaceutique utile pour le traitement des personnes atteintes du virus de l'immunodéficience humaine (VIH)**

(71) Demandeur: ASSISTANCE PUBLIQUE, HOPITAUX DE PARIS, 75004 Paris (FR)
(72) Inventeur: Leibowitch, Jacques, 75006, PARIS (FR)
(74) Mandataire: Maureau, Philippe

(57) **Abrégé**

La présente invention a pour objet une composition pharmaceutique pour le traitement du virus de l'immunodéficience humaine (VIH) chez l'être humain, comprenant quatre principes actifs choisis comme étant :
- un inhibiteur non nucléosidique de la transcriptase inverse (INNTI) choisi parmi la nevirapine, l'efavirenz et l'etravirine ;
- un inhibiteur nucléosidique de la transcriptase inverse (INTI) choisi parmi la lamivudine et l'emtricitabine ; et
- deux inhibiteurs nucléosidique ou nucléotidique de la transcriptase inverse (INTI) différents choisis parmi la didanosine, l'abacavir et le tenofovir.

## Description

La présente invention a pour objet une nouvelle composition pharmaceutique utile pour le traitement des personnes affectées par le Virus de l'Immunodéficience Humaine (VIH), responsable du Syndrome d'ImmunoDéficience Acquise (SIDA).

Le Virus de l'Immunodéficience Humaine (ou VIH) est un rétrovirus du genre des lentivirus, c'est-à-dire un virus avec une longue période d'incubation, ce qui implique une évolution lente de la maladie.

Comme tous les virus, le VIH est incapable de se multiplier tout seul. Il doit préalablement pénétrer dans une cellule et en prendre le contrôle. Les cellules cibles du VIH sont celles présentant des récepteurs CD4 à leur surface. Ainsi, les lymphocytes TCD4+, les macrophages, les cellules dendritiques et les cellules microgliales cérébrales peuvent être infectées par le VIH.

Lorsque le VIH infecte une cellule cible, il en prend contrôle. Alors, le virus commence à fabriquer de nouvelles copies de lui-même : c'est la phase de reproduction ou de réplication. Les virions ainsi produits infectent d'autres cellules. Faute de traitement, les experts estiment que le VIH peut fabriquer jusqu'à 10 milliards de copies virales chaque jour.

Deux sérotypes du VIH ont été identifiés à ce jour, le VIH-1, qui est présent dans la plupart des pays du monde, et le VIH-2 qui est principalement présent en Afrique de l'Ouest.

Il est communément admis que la réplication du virus se déroule en plusieurs étapes majeures :
1- Fixation ou attachement à une cellule cible
2- Fusion, pénétration et décapsidation
3- Transcription inverse
   Cette étape est spécifique aux rétrovirus : en effet, ces derniers ayant pour génome de l'ARN et non de l'ADN, une opération de transcription, "convertissant" l'ARN viral en ADN viral, seul à pouvoir être intégré dans le génome de la cellule cible, est nécessaire. Cette transcription est réalisée par l'enzyme de transcriptase inverse (TI).
4- Intégration
   L'ADN bicaténaire ainsi formé, étroitement associé à l'intégrase et d'autres composants protéiques viraux et cellulaires dans un complexe appelé complexe de pré-intégration, pénètre dans le noyau cellulaire. L'ADN s'intègre ensuite au hasard dans le génome de la cellule cible, sous l'effet de l'enzyme intégrase.
5- Formation d'un ARN messager (ARNm)
6- Epissage de l'ARNm ainsi obtenu
7- Traduction de l'ARNm
8- Maturation
9- Assemblage
   Les protéines de structure du virus (matrice, capside et nucléocapside) sont produites sous forme de polyprotéines. A la suite de l'étape de maturation, les différentes protéines sont liées entre elles et sont transportées vers la membrane de la cellule cible où elles rejoignent les glycoprotéines virales membranaires. Des ARN viraux rejoignent les protéines virales. Les protéines de structure s'assemblent pour former la capside (enveloppe de nature protéique recouvrant l'ADN ou l'ARN, l'ensemble étant désigné par nucléocapside) et la matrice, englobant cet ensemble.
10- Bourgeonnement
   La capside sort de la cellule infectée.
11- Maturation des virus
   Une protéase virale coupe les liens qui unissent les différentes protéines de structure (matrice, capside et nucléocapside). Suite à ces coupures, les virions (particules virales complètes avec leur enveloppe protéinique externe (capside) et leurs molécules d'ARN ou d'ADN à l'intérieur) deviennent alors infectieux et sont prêts à infecter de nouvelles cellules.

Une fois la séropositivité établie, le suivi régulier du patient est mis en place. Deux facteurs principaux sont habituellement surveillés afin de suivre l'évolution de la maladie :

### 1- Le taux de lymphocytes TCD4+

Le taux de lymphocytes TCD4+ est utilisé pour suivre la progression de l'infection vers le déficit immunitaire occasionné par le VIH. La numération des lymphocytes TCD4+ correspond au nombre de cellules T4 présentes dans le sang. Un taux normal chez l'Homme se situe entre 500 et 1 500 TCD4+/mm³ de sang. Il a été généralement admis que :
- jusqu'à 500 TCD4+/mm³ de sang, le patient pouvait vivre dans des conditions normales sans nécessiter de traitement ;
- à partir de 350 TCD4+/mm³ de sang, une proposition de traitement antiviral se discute, le résultat attendu étant le contrôle de l'activité de reproduction du VIH, et, secondairement, une remontée au moins partielle du taux de TCD4+ ;
- en dessous de 200 TCD4+/mm³ de sang, le patient est considéré comme immunodéprimé, courant le risque de souffrir de maladies définissant un SIDA avéré. Le traitement antiviral avec ou sans antibioprophylaxie s'impose comme seul capable d'éviter ces complications.

### 2- La charge virale

La concentration des particules virales HIV dans un volume de sang donne une estimation objective du nombre total de virions fraîchement produits par l'organisme du sujet infecté. La mesure est faite selon des techniques standardisées peu variables d'un laboratoire à l'autre s'il utilise ces techniques validées. Le résultat est donné en log10 du nombre de copies/ml. L'erreur sur la quantification (nombre de copies du virus) est telle qu'une variation inférieure ou égale à 0,5 est dite non significative.
La différence entre deux mesures de charge virale espacées dans le temps permet d'évaluer le taux de reproduction du VIH et donc l'évolution de l'infection. Il est généralement admis qu'il existe des liens entre la charge virale et le niveau du déficit immunitaire, manifesté par la disparition des lymphocytes TCD4+.

La charge virale est, à la date de la présente invention, le meilleur indicateur de l'évolution du virus chez le patient ; l'état des connaissances permet d'avancer en outre qu'un patient dont la charge virale plasmatique est inférieure à 50 copies/ml peut être considéré comme « non transmetteur d'infection» par voie muqueuse.

A la date de la présente invention, aucune composition pharmaceutique ne permet d'éradiquer définitivement le VIH chez une personne ayant contracté le virus, mais certaines compositions permettent de réprimer la réplication du VIH, un contrôle attesté par le maintien d'une charge virale constamment inférieure à 50 copies/ml de plasma. Ce contrôle permet d'arrêter la progression de la maladie vers un SIDA, et assure une espérance de vie pour le porteur de VIH correctement traité proche ou égale de celle des personnes de même âge et de même sexe.

Depuis le début des années 1980, de nombreuses recherches ont aboutit à l'identification d'un grand nombre d'antirétroviraux ayant pour fonction d'interférer et de bloquer les différents mécanismes nécessaire à la réplication du virus VIH, en ciblant plus particulièrement tel ou tel enzyme du VIH nécessaire à sa réplication ou en affectant les mécanismes physico chimiques présidant à l'entrée du virus dans la cellule cible.

A la date de la présente invention, les antirétroviraux constituent les seuls médicaments utilisés utilement contre le VIH. L'objectif premier et principal de cette thérapie, notamment chez le patient naïf de tout traitement, est de maintenir la charge virale en dessous du seuil de détection de 50 copies/ml de plasma aussi longtemps que possible, faute de quoi la thérapie antivirale risque de perdre son efficacité dans la durée, du fait de l'émergence de virus résistants aux drogues antivirales administrées (Hammer SM, Saag MS, Schechter M, et al., Treatment for adult HIV infection: 2006 recommendations of the International AIDS Society-USA panel. Top HIV Med (2006) 14:827-43)

Les médicaments anti VIH sont regroupés dans quatre grandes classes d'antirétroviraux, différant par leur mode d'action sur le virus HIV et contre sa reproduction et ou sa propagation dans l'organisme du porteur :
On distingue tout d'abord les inhibiteurs de la transcriptase inverse, qui inhibent le recopiage de l'ARN viral en ADN proviral, première étape dans la réplication du virus à partir de l'ARN viral. Dans cette classe, on distingue :
   - les inhibiteurs nucléosidiques ou nucléotidiques de la transcriptase inverse (INTI) ; et
   - les inhibiteurs non nucléosidiques (INNTI)
Les INTI correspondent à la première classe d'antirétroviraux mis sur le marché. Comme exemples de composés INTI, on peut citer la zidovudine (AZT, *Retrovir*®*)* et la stavudine (d4T, *Zerite*®*)* (deux analogues de la thymidine), la didanosine (ddI, *Videx*®*),* l'abacavir (ABC, *Ziagen*®*)* et le tenofovir (TDF, *Viread*®*)* (trois analogues de l'adénosine), et la lamivudine (3TC, *Epivir*®) et l'emtricitabine (FTC, *Emtriva*®) (deux analogues de la cytosine).
Les INNTI sont des inhibiteurs sélectifs et puissants de la transcriptase inverse du VIH. Comme exemples de composés INNTI on peut citer la nevirapine (NVP, *Viramune*®), l'etravirine (ETV, *Intelence*®), et l'efavirenz (EFV, *Sustiva*®). Ils ne sont actifs que sur les VIH-1.

On distingue ensuite des inhibiteurs de la protéase HIV (IP) qui agissent en inhibant l'action de l'enzyme qui dirige le découpage exact des protéines virales précurseurs de structures nécessaires à la formation du matériel HIV infectieux, et notamment les virions HIV, matériels capables de se propager dans l'organisme pour infecter de nouvelles cellules permissives. Sous l'action des inhibiteurs de la protéase HIV, on obtient des pseudos virions incapables d'infecter de nouvelles cellules. Comme exemples de composés IP, on peut citer, dans l'ordre historique de leur mise sur le marché, saquinavir (SQV, *Invirase*®), ritonavir (RTV, *Norvir*®), indinavir (IDV, *Crixivan*®), amprenavir (APV, *Agenerasec*), nelfinavir (NFV, *Viracept*®), atazanavir (ATZ, *Reyataz*®), fosamprenavir (FPV, *Telzir*®), tipranavir (TPV, *Aptivus*®), et darunavir (DRV, *Prezista*®).
Chacune de ces IP a pour propriété pharmacocinétique de s'éliminer de l'organisme du patient avec rapidité par la voie des cytochromes P450 ; le blocage partiel de cette voie d'élimination par un produit comme le ritonavir, inhibiteur puissant des fonctions cytochrome P450, prolonge sensiblement la durée de vie pharmaceutique de l'IP prescrite. Le ritonavir donné à doses faibles « booste » l'anti protéase HIV administrée dans le même temps au patient, dans la mesure où il en augmente les taux dans le sang, et en prolonge la demi-vie utile dans l'organisme.

On distingue encore les inhibiteurs d'intégrase qui bloquent l'action d'un enzyme du VIH dont la fonction élective est d'élaguer les extrémités des ADN proviraux HIV de façon à rendre cet ADN apte à servir de matrice à la transcription de l'ADN proviral en ARN HIV. Les inhibiteurs de l'intégrase rendent cet enzyme momentanément inapte à sa fonction d'élagage ADN, empêchant par là la reproduction du génome viral dans sa cellule cible. Comme exemples de composés inhibiteurs d'intégrase on peut citer le raltégravir et l'elvitégravir (GS 9137).

On distingue enfin les inhibiteurs de fusion-lyse qui interviennent avant le début du cycle biochimique de réplication du VIH, en bloquant la marche infectieuse du VIH au niveau de certaines protéines présentes à la surface des virions, ou en interférant avec les capacités de liaisons de ces protéines de surface avec des co-récepteurs présents eux-mêmes en surface de cellules cibles du VIH. Comme exemples de composés inhibiteurs de fusion-lyse, on peut citer l'enfuvirtide (*Fuzeon*®) et le maraviroc (*Celsentri*®).

Administrés seuls, la plupart des agents antirétroviraux se sont montrés seulement partiellement efficaces, incapables le plus souvent de bloquer suffisamment la reproduction du VIH pour obtenir une réduction optimale de la charge virale ou empêcher sa remontée

Pour palier à cette déficience, de nombreuses multithérapies, et en particulier des trithérapies, ont été mises au point au fil des années.

La trithérapie consiste en la co-administration de trois agents antirétroviraux, se présentant soit sous la forme de trois médicaments distincts administrés séparément, soit sous la forme d'une forme pharmaceutique unitaire contenant les trois principes actifs.

Grâce à ces multithérapies, et en particulier aux trithérapies utilisées depuis 1996, le taux de mortalité dû au SIDA a été réduit de façon significative

Sur la base de leur efficacité démontrée, et de leur acceptabilité, les combinaisons antirétrovirales préférées pour le démarrage d'une thérapie anti-VIH chez les patients vierges de traitement antérieurs ont pour socle des combinaisons de deux INTI associées soit à un IP boosté par le ritonavir, soit un INNTI (Gazzard B. British HIV Association (BHIVA) guidelines for the treatment of HIV-infected adults with antiretroviral therapy (2006), HIV Med (2006) 7:487-503).

Exceptionnellement un troisième inhibiteur de la transcriptase inverse est ajouté à la combinaison constituée d'un couple de nucléosides et d'un INNTI pour former une quadrithérapie, mais celles-ci, de même que les trithérapies associant trois INTI n'ont généralement pas été validées.

Cependant, nombre des trithérapies disponibles à la date de la présente invention sont marquées d'échappements virologiques, c'est-à-dire une charge virale chez le patient supérieure à 100 copies/ml de plasma mesurée lors deux dosages consécutifs rapprochés ; le taux des « fuites virales » s'élevant avec les années de prises ininterrompues. Dans ces cas, le taux d'échappements s'élève à 10% et plus des patients traités au bout de seulement 48 semaines de traitement, et peut passer les 20% voire les 30% au terme de 3 ou 4 années de traitements ininterrompus. Ces échappements sont la marque de combinaisons antivirales sous-optimales, et mettent en avant autant de situations dans lesquelles peut survenir une sélection de virus HIV portant des mutations de résistance au moins partielle aux composants médicamenteux de la combinaison (*First-line antiretroviral therapy with efavirenz or lopinavir*/*ritonavir plus two nucleoside analogues: the SUSKA study, a non-randomized comparison from the VACH cohort,* Pere Domingo et al., Journal of Antimicrobial Chemotherapy (2008) 61, 1348-1358). C'est le cas en particulier de la plupart des trithérapies associant seulement trois composants inhibiteurs de la transcriptase inverse et des trithérapies combinant deux composants inhibiteurs de la transcriptase inverse à la nevirapine. (Risk of Early Virological Failure of Once-Daily Tenofovir-Emtricitabine plus Twice-Daily Nevirapine in Antiretroviral Therapy-Naive HIV-Infected Patients, Giuseppe Lapadula, Silvia Costarelli, Eugenia Quiros-Roldan, et al, Clinical Infectious Diseases 2008, 46:1127-1129 ; et High rate of early virological failure with the once-daily tenofovir/lamivudine/nevirapine combination in naive HIV-1-infected patients- authors'response, D. Rey, B. Hoen, P. Chavanet, et al, J. Antimicrob. Chemother 2009; 63: 1080-1081).

D'autre part, de nombreux effets secondaires indésirables sont associés à l'utilisation de ces médicaments, parmi lesquels l'acidose lactique se caractérisant par une respiration profonde et rapide, une somnolence, des nausées, des vomissements et/ou des douleurs d'estomac ; des sensations de vertiges ; des difficultés à dormir ; des difficultés à se concentrer ; des rêves anormaux ; des éruptions cutanées ; des inflammations ou des infections diverses ; et/ou des problèmes osseux...

Enfin, la nécessité d'administrer au patient le traitement de façon journalière sept jours par semaine fait de ces thérapies des traitements lourds et contraignants pour le patient, et tend à augmenter l'intensité des effets secondaires ressentis par celui-ci. De fait, les patients sont souvent conduits à ne pas observer strictement les recommandations en termes d'administration de la thérapie.

Or, selon le Professeur Jean-Francois Delfraissy, c'est la non-observance du traitement qui est la principale cause de l'échec thérapeutique (« Prise en charge thérapeutique des personnes infectées par le VIH - Rapport 2004 - Sous la direction du Professeur Jean-François Delfraissy », 2004, Éditions Flammarion, p. 48-49).

A la date de la présente invention, deux trithérapies (Trizivir® un médicament mis sur le marché par le laboratoire pharmaceutique Glaxo Smith Kline et Atripla®, un médicament mis sur le marché par le laboratoire pharmaceutique Gilead) permettent l'administration journalière sept jours par semaine du traitement sous la forme d'une forme posologique unitaire.
Trizivir® se présente sous la forme d'un unique comprimé pelliculé comprenant :
- 150 mg de lamivudine ;
- 300 mg de zidovudine ; et
- 300 mg d'abacavir base (351 mg de sulfate d'abacavir).
Atripla® se présente sous la forme d'un unique comprimé pelliculé comprenant :
- 600 mg d'efavirenz ;
- 200 mg d'emtricitabine ; et
- 245 mg de tenofovir disoproxil fumarate (exprimé en tenofovir disoproxil).
Cette seconde composition pharmaceutique, qui est parmi les trithérapies les plus efficaces actuellement présentes sur le marché, nécessite néanmoins une administration journalière sept jours par semaine, ce qui est loin de favoriser la meilleure observance du traitement par les patients.
Ni Atripla®, ni Trizivir® n'ont permis une diminution des effets secondaires mentionnés ci-dessus.

De plus, conformément aux Directives émises par la Food and Drug Administration (FDA) en 2008 (FDA Guidelines 2008), l'efavirenz ne peut être utilisé chez la femme enceinte. Ainsi, Atripla®, qui contient de l'efavirenz ne peut, par extension, être utilisé chez la femme enceinte, ce qui constitue évidemment un sérieux inconvénient.

Enfin, le coût par patient et par an des multithérapies existantes à la date de la présente invention demeure excessivement élevé. Ainsi, à titre d'exemple, Atripla® est vendu en France sous forme flacon contenant 30 comprimés (soit un mois de traitement) pour le prix de 834,30€, soit un coût annuel par patient d'environ 10.000€. Or, si les traitements actuels permettent de fortement limiter le développement du virus VIH chez les patients atteints, ils ne permettent en aucun cas de l'éradiquer. Le coût du traitement des personnes affectées par le VIH peut donc atteindre des montants très importants, amenés à augmenter fortement dans le futur.

Ainsi, il demeure plus que jamais nécessaire de mettre au point des multithérapies alternatives qui permettent de limiter le nombre d'administrations du traitement au patient pour assurer une meilleure observance par celui-ci, limiter les effets secondaires associés au traitement-ci et diminuer les coûts associés à ce dernier, tout en maintenant une efficacité thérapeutique au moins comparable à celle des multithérapies existantes, et en particulier Atripla®.

D'autres multithérapies ont ainsi été testées depuis la mise sur le marché d'Atripla®. A titre d'exemple on peut citer l'étude rapportée par la société Boehringer Ingelheim Pharmaceuticals dans le *Biotech Finances* en date du 20 juillet 2009. Cette publication mentionne les résultats d'une étude comparative menée sur deux trithérapies : l'une consistant à administrer la nevirapine associée au tenofovir et à l'emtricitabine, et l'autre consistant à administrer l'atazanavir/ritonavir associé au tenofovir et à l'emtricitabine. Chacune de ces associations a été administrée une fois par jour. De plus, cette étude a fait ressortir qu'une augmentation de la charge virale était observée après 48 semaines de traitement chez près de 35% des patients traités.

Ferrer E, Gatell JM, Sanchez P, Domingo P et al. ont rapporté dans AIDS Res Hum Retroviruses, 2008-07 ;24(7):931-4 l'évaluation d'une quadrithérapie associant la zidovudine, la lamivudine l'abacavir et le tenofovir, soit quatre INTI, à doses standard lors d'un essai pilote prospectif non contrôlé de 96 semaines chez des adultes atteints du HIV et vierges de traitement antérieurs. Seuls 34 sur 39 des patients traités (87%) ont attient une charge virologique inférieure ou égale à 50 copies/ml à la 96ème semaine, ce qui représente 13% d'échecs virologique. Cette publication ne fait aucune mention d'une quadrithérapie associant 3 INTI à un INNTI, ni à une éventuelle diminution du nombre d'administration hebdomadaire de ladite quadrithérapie ainsi évaluée.

Il a maintenant été trouvé, de façon inattendue, une nouvelle composition pharmaceutique pour le traitement du VIH qui permette à la fois de diminuer le nombre d'administrations au patient tout en maintenant une efficacité au moins comparable à celle des multithérapies existantes, et en particulier Atripla®.

La présente invention a donc pour objet une composition pharmaceutique pour le traitement du virus de l'immunodéficience humaine (VIH) chez l'être humain, comprenant quatre principes actifs choisis comme étant :
- un inhibiteur non nucléosidique de la transcriptase inverse (INNTI) choisi parmi la nevirapine, l'efavirenz et l'etravirine ;
- un inhibiteur nucléosidique de la transcriptase inverse (INTI) choisi parmi la lamivudine et l'emtricitabine ; et
- deux inhibiteurs nucléosidique ou nucléotidique de la transcriptase inverse (INTI) différents choisis parmi la didanosine, l'abacavir et le tenofovir.

La composition pharmaceutique selon l'invention permet non seulement de diminuer le nombre d'administrations au patient, tout en maintenant une efficacité au moins comparable à celle des multithérapies existantes, et en particulier Atripla®. De plus, la composition selon l'invention est également susceptible d'être administrée à la femme enceinte, ce qui permet, entre autres choses, une poursuite du traitement dès les premiers mois d'une grossesse, même inattendue.

Ces résultats sont d'autant plus inattendus que la didanosine est un agent antirétroviral connu pour avoir une faible capacité à empêcher à lui seul le recopiage du VIH (« puissance antivirale intrinsèque » faible). Les auteurs Jemsek J, Hutcherson P, et Harper E. rapportent d'ailleurs dans leur article "Poor virologic responses and early emergence of resistance in treatment naive, HIV-infected patients receiving a once daily triple nucleoside regimen of didanosine, lamivudine, and tenofovir". (DF. 11 th Conference on Retroviruses and Opportunistic Infections, San Francisco, 8-11 février 2004, Abstract 51) un taux d'échec virologique précoce de 91% lors de l'utilisation de la trithérapie associant la didanosine, le tenofovir et la lamivudine.
De plus, les études rapportées par Agathe Leon, Esteban Martinez, Josep Mallolas, et al, Early virological failure in treatment-naïve HIV-infected adults receiving didanosine and tenofovir plus efavirenz or Nevirapine (AIDS 2005, 19:209-215) ; et Jose R. Arribas, The rise and fall of triple nucleoside reverse transcriptase inhibitor regimens (Journal of Antimicrobial Chemotherapy (2004) 54, 587-592) ont montrées un taux d'échec virologique précoce très important (50% ou plus) lors de l'utilisation de trithérapies associant le tenofovir et la didanosine à un INNTI tel que l'efavirenz ou la nevirapine.

Dans le cadre de la présente invention :
- VIH désigne exclusivement le VIH-1 ;
- on entend par « sel pharmaceutiquement acceptable » d'un principe actif tout sel d'addition dudit principe actif avec un acide minéral ou organique par action d'un tel acide au sein d'un solvant organique ou aqueux tel qu'un alcool, une cétone, un éther ou un solvant chloré, et qui soit acceptable d'un point de vue pharmaceutique ;

- on entend par « dérivé pharmaceutiquement acceptable » d'un principe actif tout «prodrogue» ou «métabolite» dudit principe actif, ainsi que leur sel pharmaceutiquement acceptable ;
- on entend par « prodrogue » d'un principe actif tout composé dont la biotransformation dans l'organisme aboutit audit principe actif ;
- on entend par « métabolite » d'un principe actif tout produit intermédiaire résultant de la transformation dudit principe actif dans l'organisme lors d'un processus métabolique ;
- on entend par « administration journalière » une administration une fois par jour ou une administration une fois par 24 heures ;
- on entend par « calendrier continu » le traitement thérapeutique continu d'un patient, comprenant l'administration successive d'une ou plusieurs compositions thérapeutiques (dont multithérapies, selon l'invention ou non), identiques ou différentes, chacune avec son propre schéma d'administration thérapeutique (nombre d'administrations journalières et nombre de jours d'administration sur une période donnée, semaine par exemple) et ce, de façon illimitée et non séquencée ou espacée dans le temps, c'est-à-dire sans interruption de traitement ;
- la nevirapine (ou NVP) désigne la 11-cyclopropyl-5,11-dihydro-4-methyl- 6H-dipyrido[3,2-b:2',3'-f][1,4]diazepin-6-one, ainsi que sessels ou dérivés pharmaceutiquement acceptables ;
- l'efavirenz (ou EFV) désigne la (S)-6-chloro-4-(cyclopropylethynyl) -1,4-dihydro-4-(trifluoromethyl) -2H-3,1-benzoxazin-2-one, ainsi que sessels ou dérivés pharmaceutiquement acceptables ;
- l'etravirine (ou ETV) désigne le 4-({6-amino-5-bromo-2 -[(4-cyanophenyl)amino]pyrimidin-4-yl}oxy) -3,5-dimethylbenzonitrile, ainsi que ses sels ou dérivés pharmaceutiquement acceptables ;
- la lamivudine (ou 3TC) désigne la (2*R,5S*)-(-)-4-amino-1-[2-(hydroxymethyl)-1,3 - oxathiolan-5-yl]-1H-pyrimidin-2-one, ainsi i que e sessels ou dérivés s pharmaceutiquement acceptables ;
- l'emtricitabine (ou FTC) désigne la L-2',3'-didéoxy-5-fluoro-3'-thiacytidine, ainsi que ses sels ou dérivés pharmaceutiquement acceptables ;
- la didanosine (ou DDI) désigne la L-2',3'-didéoxyinosine, ainsi que ses sels ou dérivés pharmaceutiquement acceptables ;
- l'abacavir (ou ABC) désigne le [(1S,4R)-4-[2-amino-6-(cyclopropylamino)-9H-purin-9-yl]cyclopent-2-ényl]méthanol, ainsi que ses sels ou dérivés pharmaceutiquement acceptables, parmi lesquels l'abacavir sulfate ; et
- le tenofovir (ou TDF) désigne le L (R)-9-(2-phosphonylméthoxy-propyl)adénine, ainsi que ses sels ou dérivés pharmaceutiquement acceptables, parmi lesquels le tenofovir disoproxil ou le fumarate de tenofovir disoproxil.

Préférentiellement, la présente invention a pour objet une composition pharmaceutique telle que définie ci-dessus, dans laquelle les caractéristiques suivantes sont choisies seules, ou en combinaison :
- l'INNTI est choisi comme étant la nevirapine ;
- le « premier » INTI est choisi comme étant l'emtricitabine ;
- les deux autres INTI sont choisis comme étant le tenofovir et la didanosine.

De façon toute à fait préférée, la présente invention a pour objet une composition pharmaceutique comprenant, à titre de principes actifs, la nevirapine, l'emtricitabine, le tenofovir et la didanosine.

Comme autres exemples de compositions pharmaceutiques selon la présente invention, on peut notamment citer les compositions pharmaceutiques comprenant :
- la nevirapine, l'emtricitabine, l'abacavir, et la didanosine ;
- la nevirapine, la lamivudine, l'abacavir et la didanosine ;
- la nevirapine, la lamivudine, le tenofovir et la didanosine ;
- l'efavirenz, l'emtricitabine, l'abacavir, et la didanosine ;
- l'efavirenz, l'emtricitabine, le tenofovir et la didanosine ;
- l'efavirenz, la lamivudine, l'abacavir et la didanosine ;
- l'efavirenz, la lamivudine, le tenofovir et la didanosine ;
- l'etravirine, l'emtricitabine l'abacavir, et la didanosine ;
- l'etravirine, l'emtricitabine, le tenofovir et la didanosine ;
- l'etravirine, la lamivudine, l'abacavir et la didanosine ; et
- l'etravirine, la lamivudine, le tenofovir et la didanosine.

La composition pharmaceutique selon la présente invention contient les principes actifs en quantité suffisante pour assurer l'effet thérapeutique souhaité, c'est-à-dire le traitement du VIH en maintenant chez le patient traité une charge virale inférieure à 50 copies/ml, de préférence inférieure ou égale à 20 copies/ml.

Le cas échéant, la composition pharmaceutique selon la présente invention permet également le maintien ou la remontée du taux de lymphocytes TCD4+ à un niveau de préférence supérieur aux taux de TCD4+/mm³ du patient avant son traitement antiviral par la composition de l'invention.

De préférence, les quantités suivantes d'agents antirétroviraux sont utilisées pour préparer la composition pharmaceutique selon l'invention :
- de 100 à 300 mg d'emtricitabine, de préférence 200 mg d'emtricitabine ;
- de 200 à 400 mg de lamivudine, de préférence 300 mg de lamivudine ;
- de 500 à 700 mg d'abacavir, de préférence 600 mg d'abacavir ;
- de 100 à 300 mg de tenofovir, de préférence 245 mg de tenofovir ;
- de 150 à 350 mg de didanosine, de préférence 250 mg de didanosine ;
- de 300 à 500 mg de nevirapine, de préférence 400 mg de nevirapine ;
- de 300 à 500 mg d'etravirine, de préférence 400 mg d'etravirine ;
- de 100 à 700 mg d'efavirenz, de préférence 200, 400 ou 600 mg d'efavirenz.

La composition pharmaceutique selon la présente invention peut être formulée sous toute forme galénique nécessaire à son administration. En particulier, s'agissant d'administration par voie orale, les compositions selon la présente invention peuvent être formulées sous forme de comprimés enrobés ou non, effervescents, solubles, orodispersibles, gastrorésistants ou à libération modifiée ; de dragées ; de capsules à enveloppe dure (ou gélules) ; de capsules à enveloppe molle ; de granules ; de granulés ; de pilules ; de pastilles. S'agissant d'administration par voie systémique, la composition selon l'invention peut être formulée sous forme de poudre lyophilisée stérile pour injection. Les compositions pharmaceutiques selon la présente invention pourront donc comprendre, en plus des principes actifs, tout adjuvant de formulation pharmaceutiquement acceptable, connu de l'homme du métier et qui est nécessaire à la préparation de la composition pharmaceutique sous la forme souhaitée.

Certaines compositions pharmaceutiques selon la présente invention peuvent être administrées à tout patient contaminé par le VIH, y compris la femme enceinte. Ainsi, la présente invention a également pour objet une composition pharmaceutique telle que définie précédemment et dans laquelle l'INNTI est choisi parmi la nevirapine et l'etravirine, pour le traitement du virus de l'immunodéficience humaine (VIH) chez la femme enceinte.

La composition pharmaceutique selon la présente invention permet de diminuer le nombre d'administration au patient tout en maintenant une efficacité au moins comparable à celle des multithérapies existantes, et en particulier Atripla®. Ainsi, la présente invention a également pour objet une composition pharmaceutique telle que définie précédemment pour une administration journalière un à sept jours par semaine, de préférence un à six jours par semaine, de préférence encore deux à quatre jours par semaine, à un être humain affecté par le VIH.

La composition pharmaceutique selon l'invention peut être administrée à tout moment de la journée, avant, pendant ou après les repas, sans que cela n'influe sur l'efficacité du traitement.

La composition selon la présente invention peut être administrée selon un calendrier continu.

Un autre objet de la présente invention concerne également l'utilisation d'une composition pharmaceutique telle que définie précédemment pour la préparation d'un médicament destiné au traitement du VIH chez l'être humain.

Un autre objet de la présente invention concerne également l'utilisation d'une composition pharmaceutique telle que définie précédemment et dans laquelle l'INNTI choisi parmi la nevirapine et l'etravirine pour la préparation d'un médicament destiné au traitement du VIH chez la femme enceinte.

Un autre objet de la présente invention concerne également l'utilisation d'une composition pharmaceutique telle que définie précédemment pour la préparation d'un médicament destiné au traitement du VIH chez l'être humain, ledit médicament étant administré de façon journalière un à sept jours par semaine, de préférence un à six jours par semaine, de préférence encore deux à quatre jours par semaine audit être humain, l'administration pouvant ou non s'effectuer selon un calendrier continu.

La présente invention a également pour objet une méthode de traitement du VIH chez un être humain affecté par ce virus par l'administration d'une composition pharmaceutique telle que de définie précédemment.

La présente invention a également pour objet une méthode de traitement du VIH chez la femme enceinte affectée par ce virus par l'administration d'une composition pharmaceutique telle que de définie précédemment et dans laquelle l'INNTI choisi parmi la nevirapine et l'etravirine.

La présente invention a également pour objet une méthode de traitement du VIH chez un être humain affecté par ce virus par l'administration journalière un à sept jours par semaine, de préférence un à six jours par semaine, de préférence encore deux à quatre jours par semaine, d'une composition pharmaceutique telle que de définie précédemment, l'administration pouvant ou non s'effectuer selon un calendrier continu.

Les quatre principes actifs constituant la nouvelle multithérapie selon l'invention peuvent être administrés sous la forme d'une composition pharmaceutique unitaire comprenant les quatre principes actifs permettant une administration de ladite composition au patient en une seule prise.

Néanmoins, une administration séparée d'un ou plusieurs des principes actifs constitutifs de la nouvelle multithérapie selon l'invention peut également être envisagée. Ainsi, la présente invention a également pour objet un produit pharmaceutique contenant :
- un inhibiteur non nucléosidique de la transcriptase inverse (INNTI) tel que défini précédemment ; et
- trois inhibiteurs nucléosidique ou nucléotidique de la transcriptase inverse (INTI) tels que définis précédemment ;
comme produit de combinaison (ou kit pharmaceutique) pour une administration simultanée, séparée ou étalée dans le temps dans le cadre du traitement du VIH chez l'être humain.

A titre d'exemple, le produit pharmaceutique selon la présente invention peut se présenter sous la forme :
- d'une forme posologique unitaire contenant un INTI tel que défini précédemment et d'une forme posologique unitaire contenant les deux autres INTI et un INNTI tels que définis précédemment; ou
- d'une forme posologique unitaire contenant un INNTI tel que défini précédemment et d'une forme posologique unitaire contenant les trois INTI tels que définis précédemment ; ou
- d'une forme posologique unitaire contenant un INTI et un INNTI tels que définis précédemment et d'une forme posologique unitaire contenant les deux autres INTI tels que définis précédemment ;
- d'une forme posologique unitaire contenant un INTI et un INNTI tels que définis précédemment et de deux autres formes posologiques unitaires contenant chacune un INTI tel que défini précédemment ; ou
- d'une forme posologique unitaire contenant deux INTI tels que définis précédemment, d'une forme posologique unitaire contenant un INNTI tel que défini précédemment et d'une forme posologique unitaire contenant un INTI tel que défini précédemment ;
- de quatre formes posologiques unitaires contenant respectivement les quatre principes actifs selon l'invention, tels que définis précédemment.

Dans le cadre de la présente invention, la forme posologique unitaire contenant la nevirapine et la didanosine constitue une entité pharmaceutique spécifique et préférée.

Ainsi, à titre d'exemples préférentiels, on peut citer le produit pharmaceutique se présentant sous la forme ;
- d'une forme posologique unitaire contenant la nevirapine et la didanosine, et d'une forme posologique unitaire contenant l'abacavir et la lamivudine (commercialisée sous le nom Kivexa®) ;
- d'une forme posologique unitaire contenant la nevirapine et la didanosine, d'une forme posologique unitaire contenant l'abacavir, et d'une forme posologique unitaire contenant la lamivudine ;
- d'une forme posologique unitaire contenant la nevirapine et la didanosine, et d'une forme posologique unitaire contenant l'emtricitabine et le tenofovir (commercialisée sous le nom Truvada®) ;
- d'une forme posologique unitaire contenant la nevirapine et la didanosine, et d'une forme posologique unitaire contenant l'abacavir et l'emtricitabine ;
- d'une forme posologique unitaire contenant la nevirapine et la didanosine, d'une forme posologique unitaire contenant l'abacavir, et d'une forme posologique unitaire contenant l'emtricitabine ;
- d'une forme posologique unitaire contenant la nevirapine et la didanosine, et d'une forme posologique unitaire contenant l'abacavir et le tenofovir ; ou
- d'une forme posologique unitaire contenant la nevirapine et la didanosine, d'une forme posologique unitaire contenant l'abacavir, et d'une forme posologique unitaire contenant le tenofovir.

Le produit pharmaceutique selon l'invention peut bien entendu être administré selon l'un des schémas d'administration définis précédemment. Ainsi, la présente invention a également pour objet un produit pharmaceutique contenant :
- un inhibiteur non nucléosidique de la transcriptase inverse (INNTI) tel que défini précédemment ; et
- trois inhibiteurs nucléosidique ou nucléotidique de la transcriptase inverse (INTI) tels que définis précédemment ;
comme produit de combinaison (ou kit pharmaceutique) pour une administration journalière simultanée, séparée ou étalée dans le temps un à sept jours par semaine, de préférence un à six jours par semaine, de préférence encore deux à quatre jours par semaine, dans le cadre du traitement du VIH chez l'être humain.

La présente invention est illustrée de manière non limitative par les exemples suivants.

Dans les exemples suivants (exemples 1 à 1), les doses journalières de principe actif utilisées pour traiter les patients correspondent, sauf mention contraire, aux doses suivantes :
- 200 mg d' emtricitabine ;
- 300 mg de lamivudine ;
- 600 mg d'abacavir ;
- 245 mg de tenofovir ;
- 250 mg de didanosine ;
- 400 mg de nevirapine ;
- 400 mg d' etravirine ;
- 600 mg d'efavirenz.

### Exemple 1

Un patient affecté par le VIH non traité durant 36 mois a été traité selon le protocole suivant :
- traitement par une quadrithérapie associant l'efavirenz (EFV), la lamivudine (3TC), et le tenofovir (TDF) et la didanosine (DDI), administrée de façon journalière 7 jours par semaine ;
- puis un traitement par une quadrithérapie associant l'efavirenz (EFV), l'emtricitabine (FTC), et le tenofovir (TDF) et la didanosine (DDI), administrée de façon journalière 7 jours par semaine ;
- et enfin un traitement par une quadrithérapie associant la nevirapine (NVP), l'emtricitabine (FTC), et le tenofovir (TDF) et la didanosine (DDI), administrée de façon journalière 7 jours par semaine, puis 5 jours par semaine, puis 4 jours par semaine, puis 3 jours par semaine, puis 2 jours par semaine.

Durant ce traitement, la charge virale plasmatique, ainsi que le taux de TCD4+ dans le sang ont été mesurés.

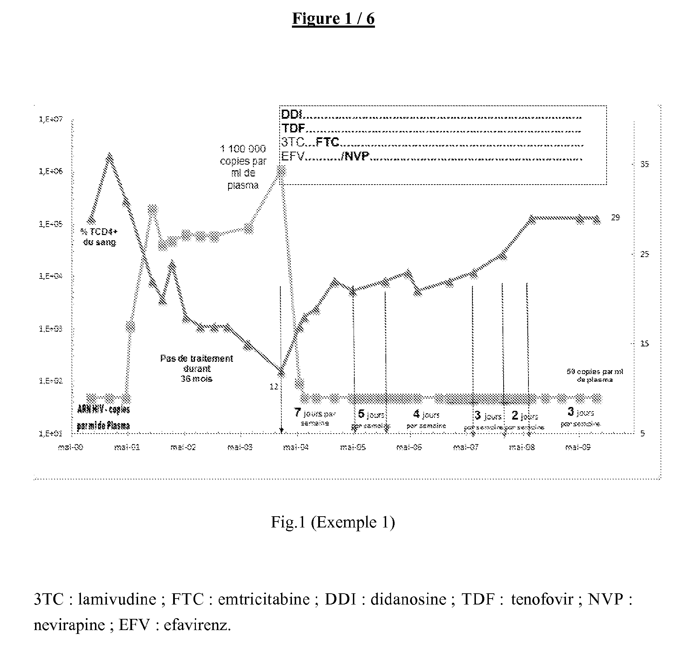

Les résultats sont rapportés dans le Figure 1.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 2

Un patient affecté par le VIH a été traité par une quadrithérapie associant la nevirapine (NVP), l'emtricitabine (FTC), et le tenofovir (TDF) et la didanosine (DDI), administrée de façon journalière 7 jours par semaine, puis 5 jours par semaine, puis 4 jours par semaine, puis 3 jours par semaine.

Durant ce traitement, la charge virale plasmatique, ainsi que le taux de TCD4+ dans le sang ont été mesurés.

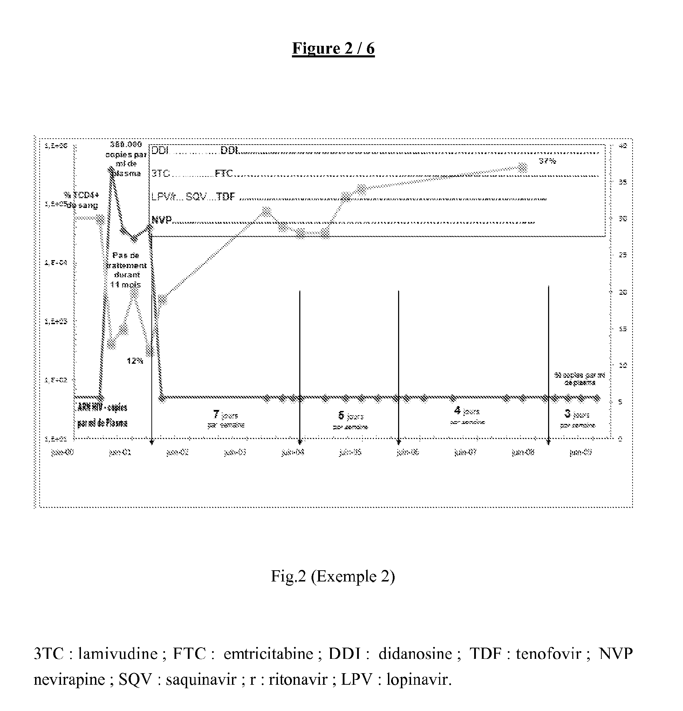

Les résultats sont rapportés dans le Figure 2.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 3

Un patient affecté par le VIH a été traité par une quadrithérapie associant la nevirapine (NVP), l'emtricitabine (FTC), et le tenofovir (TDF) et la didanosine (DDI), administrée de façon journalière 7 jours par semaine, puis 5 jours par semaine, puis 4 jours par semaine, puis 3 jours par semaine.

Durant ce traitement, la charge virale plasmatique, ainsi que le taux de TCD4+ dans le sang ont été mesurés.

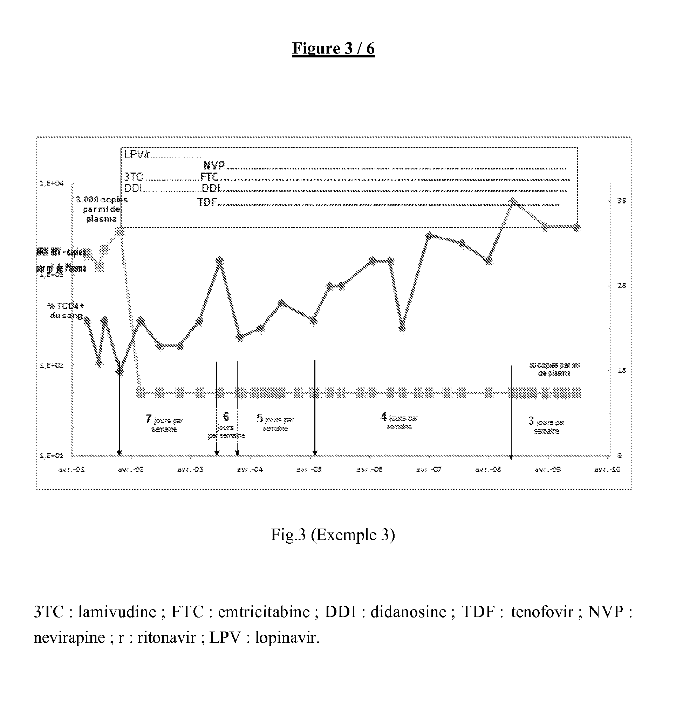

Les résultats sont rapportés dans le Figure 3.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 4

Un patient affecté par le VIH a été traité selon le protocole suivant :
- traitement par une quadrithérapie associant l'efavirenz (EFV) 600 mg, l'emtricitabine (FTC), et le tenofovir (TDF) et la didanosine (DDI), administrée de façon journalière 7 jours par semaine ;
- puis un traitement par une quadrithérapie associant l'efavirenz (EFV) 400 mg, l'emtricitabine (FTC), et le tenofovir (TDF) et la didanosine (DDI), administrée de façon journalière 5 jours par semaine, puis 4 jours par semaine, puis 3 jours par semaine, puis 2 jours par semaine.

Durant ce traitement, la charge virale plasmatique, ainsi que le taux de TCD4+ dans le sang ont été mesurés.

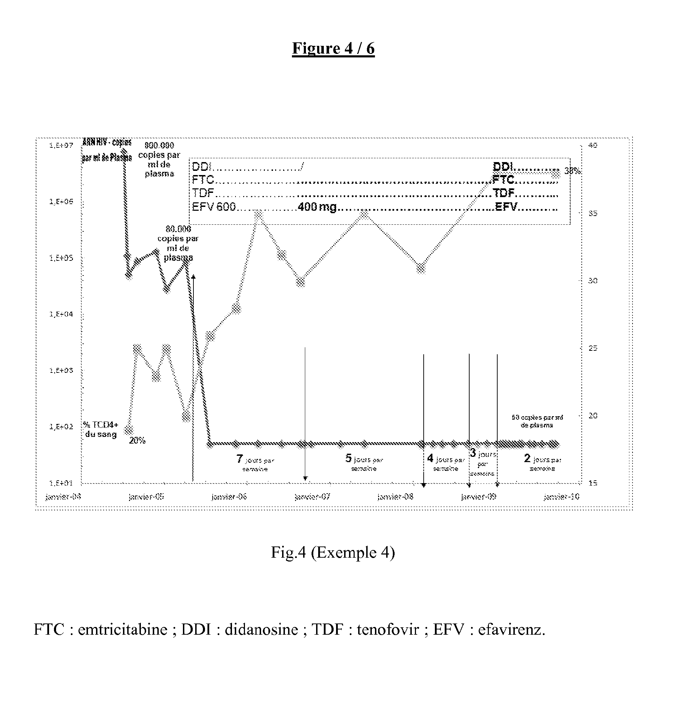

Les résultats sont rapportés dans le Figure 4.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 5

Un patient affecté par le VIH non traité durant 24 mois a été traité selon le protocole suivant :
- traitement par une quadrithérapie associant l'efavirenz (EFV), l'emtricitabine (FTC), et le tenofovir (TDF) et la didanosine (DDI), administrée de façon journalière 7 jours par semaine, puis 5 jours par semaine ;
- puis un traitement par une trithérapie ;
- et enfin un traitement par une quadrithérapie associant l'etravirine (ETV), l'emtricitabine (FTC), et le tenofovir (TDF) et la didanosine (DDI), administrée de façon journalière 3 jours par semaine.

Durant ce traitement, la charge virale plasmatique, ainsi que le taux de TCD4+ dans le sang ont été mesurés.

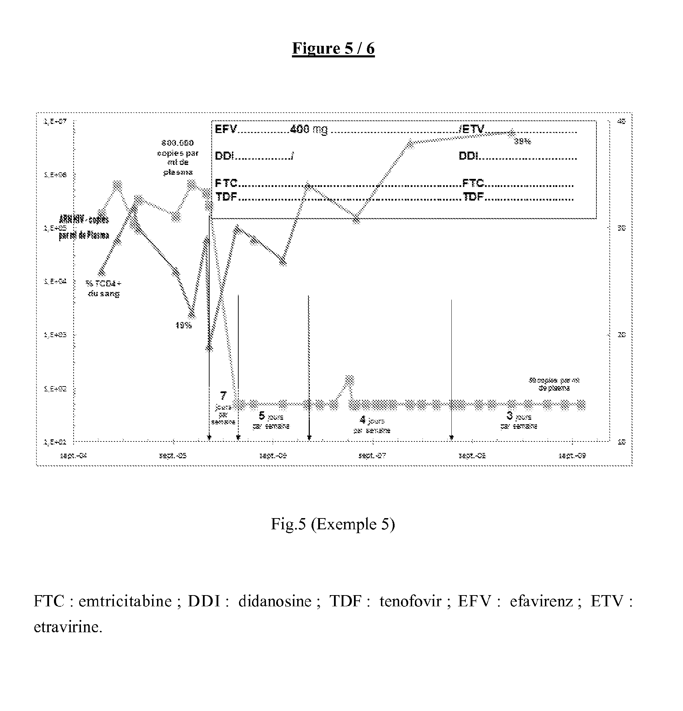

Les résultats sont rapportés dans le Figure 5.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 6

Un patient affecté par le VIH non traité durant 24 mois a été traité selon le protocole suivant :
- traitement par une quadrithérapie associant l'efavirenz (EFV) 400mg, la lamivudine (3TC), et le tenofovir (TDF) et la didanosine (DDI), administrée 7 de façon journalière jours par semaine, puis 5 jours par semaine ;
- puis un traitement par une trithérapie ;
- et enfin un traitement par une quadrithérapie associant l'efavirenz (EFV) 200mg, l'emtricitabine (FTC), et le tenofovir (TDF) et la didanosine (DDI), administrée de façon journalière 3 jours par semaine, puis 2 jours par semaine.

Durant ce traitement, la charge virale plasmatique, ainsi que le taux de TCD4+ dans le sang ont été mesurés.

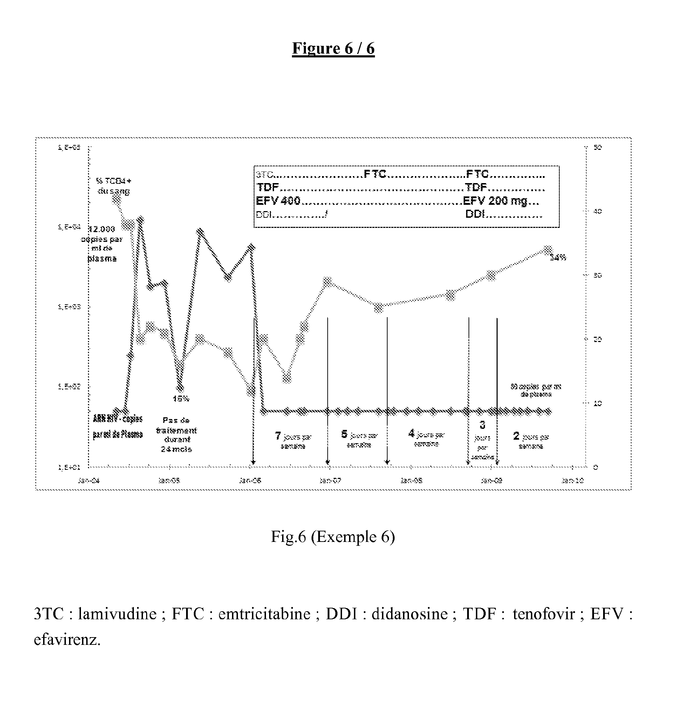

Les résultats sont rapportés dans le Figure 6.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 7

Le nombre de « blips HIV » sous traitement, c'est-à-dire les apparitions évanescentes du VIH dans le plasma au-dessus de 50 copies par ml de plasma chez les patients traités, ont été mesurés chez les patients sous traitement par Atripla® en comparaison des patients traités par les quadrithérapies selon l'invention.

Les résultats obtenus sont rapportés dans le tableau suivant.

| **Traitement** | **Efavirenz + 2 INTI (Atripla ® et autres)** (Nombre de « blips » / nombre de patients) | **Nevirapine + 3 INTI** (Nombre de « blips » / nombre de patients) | **Efavirenz + 3 INTI** (Nombre de « blips » / nombre de patients) |
|---|---|---|---|
| 7 jours / semaine | 2/61 | 0/14 | 2/55 |
| 5 jours / semaine | 1 / 70 | 0/29 | 0/40 |
| 4 jours / semaine | 4/123 | 0/48 | 0/6 |
| 3 jours / semaine | 2 / 93 dont 1 échec | 0 / 18 | 0 / 6 |
| 2 jours / semaine | 4 / 34 dont 1 échec | 0 / 10 | 0 / 10 |

Ainsi, contrairement au préjugé dominant, la réduction des quantités et du nombre d'administration des drogues antivirales chez les patients patient n'entraine pas la reprise de la réplication du VIH, à proportion inverse de la pression exercée quotidiennement par la combinaison en question.
En effet, dans la mesure où l'incidence des « blips » peut être interprété comme le reflet de l'activité réplicative du VIH couvant chez les patients les mieux traités en deçà des seuils techniques de détection, celle-ci devrait augmenter lorsque la pression antivirale diminue - soit que la combinaison antivirale ne soit plus suffisamment puissante au quotidien, ou que les prises d'antiviraux soient irrégulièrement espacées dans la semaine. Or il n'en est rien.

De plus, chez les patient traités avec la quadrithérapie associant la nevirapine, la didanosine, l'emtricitabine et le tenofovir, aucun échec ni aucun « blip » n'a été enregistré lors des 120 dosages effectués chez 7 patients ainsi traités, pendant une moyenne de 18 mois chacun (554 semaines), à raison de : cinq jours par semaine (3 patients et 110 semaines cumulées), ou quatre jours (3 patients et 270 semaines de traitement), ou trois jours (3 patients et 137 semaines), ou 2 jours par semaine (2 patients et 36 semaines de traitement antiviral intermittent).

En revanche, chez les 53 patients traités par Atripla ou équivalent sept jours par semaines comme recommandé, sur une durée moyenne de 121 semaines, l'incidence des « blips » a été de 10 sur 450 dosages.

## Revendications

1. Composition pharmaceutique pour le traitement du virus de l'immunodéficience humaine (VIH) chez l'être humain comprenant quatre principes actifs choisis comme étant :
- un inhibiteur non nucléosidique de la transcriptase inverse (INNTI) choisi parmi la nevirapine, l'efavirenz et l' etravirine ;
- un inhibiteur nucléosidique de la transcriptase inverse (INTI) choisi parmi la lamivudine et l'emtricitabine ; et
- deux inhibiteurs nucléosidique ou nucléotidique de la transcriptase inverse (INTI) différents choisis parmi la didanosine, l'abacavir et le tenofovir.

2. Composition pharmaceutique selon la revendication 1, **caractérisée en ce que** l'INNTI est la nevirapine ou l'etravirine.

3. Composition pharmaceutique selon la revendication 2, **caractérisée en ce que** l'INNTI est la nevirapine.

4. Composition pharmaceutique selon l'une quelconque des revendications 1 à 3, **caractérisée en ce que** l'un des INTI est l'emtricitabine.

5. Composition pharmaceutique selon l'une quelconque des revendications 1 à 3, **caractérisée en ce que** les trois INTI sont l'emtricitabine, le tenofovir et la didanosine.

6. Composition pharmaceutique selon l'une quelconque des revendications 1 à 5, **caractérisée en ce qu'**elle comprend, à titre de principes actifs, la nevirapine, l'emtricitabine, le tenofovir et la didanosine.

7. Composition pharmaceutique selon la revendication 6, **caractérisée en ce qu'**elle comprend 400 mg de nevirapine, 200 mg d'emtricitabine, 245 mg de tenofovir et 250 mg de didanosine

8. Composition pharmaceutique selon l'une quelconque des revendications 2 à 7, pour le traitement de la femme enceinte.

9. Composition pharmaceutique selon l'une quelconque des revendications 1 à 7, pour une administration journalière un à six jours par semaine.

10. Composition pharmaceutique selon la revendication 9, pour une administration journalière deux à quatre jours par semaine.

11. Produit pharmaceutique contenant :
- un inhibiteur non nucléosidique de la transcriptase inverse (INNTI) tel que défini dans l'une des revendications 1 à 3; et
- trois inhibiteurs nucléosidique ou nucléotidique de la transcriptase inverse (INTI) tels que définis dans l'une des revendications 1, 4 ou 5 ;
comme produit de combinaison pour une administration simultanée, séparée ou étalée dans le temps dans le cadre du traitement du VIH chez l'être humain.

12. Produit pharmaceutique selon la revendication 11 se présentant sous la forme d'une forme posologique unitaire contenant la nevirapine et la didanosine, et d'une forme posologique unitaire contenant l'abacavir et la lamivudine.

13. Produit pharmaceutique selon la revendication 12 se présentant sous la forme d'une forme posologique unitaire contenant la nevirapine et la didanosine, et d'une forme posologique unitaire contenant l'emtricitabine et le tenofovir.
